(19) Europäisches Patentamt / European Patent Office / Office européen des brevets

(11) **EP 3 021 757 B1**

(12) **EUROPEAN PATENT SPECIFICATION**

(45) Date of publication and mention
of the grant of the patent:
**26.08.2020  Bulletin 2020/35**

(21) Application number: **13771245.1**

(22) Date of filing: **17.07.2013**

(51) Int Cl.:
*A61B 8/00* (2006.01)    *A61B 5/00* (2006.01)
*A61B 5/053* (2006.01)

(86) International application number:
**PCT/TR2013/000225**

(87) International publication number:
**WO 2015/009251 (22.01.2015 Gazette 2015/03)**

(54) **MULTIFREQUENCY ELECTRICAL IMPEDANCE IMAGING USING LORENTZ FIELDS**

MULTIFREQUENTE ELEKTRISCHE IMPEDANZBILDGEBUNG MIT LORENTZ-FELDERN

IMAGERIE D'IMPÉDANCE ÉLECTRIQUE MULTIFRÉQUENCE UTILISANT LES CHAMPS DE LORENTZ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**25.05.2016  Bulletin 2016/21**

(73) Proprietors:
  • **Gencer, Nevzat Guneri**
    **06800 Ankara (TR)**
  • **Zengin, Reyhan**
    **Battalgazi/Malatya (TR)**

(72) Inventors:
  • **Gencer, Nevzat Guneri**
    **06800 Ankara (TR)**
  • **Zengin, Reyhan**
    **Battalgazi/Malatya (TR)**

(74) Representative: **Kayahan, Senem et al**
    **Yalciner Patent and Consulting Ltd.**
    **Tunus Cad. No: 85/3-4**
    **Kavaklidere Cankaya**
    **06680 Ankara (TR)**

(56) References cited:
    **WO-A1-98/00732     CN-A- 102 860 825**

• HUI XIA ET AL: "Experimental study of magneto-acousto-electrical tomography", MECHANIC AUTOMATION AND CONTROL ENGINEERING (MACE), 2011 SECOND INTERNATIONAL CONFERENCE ON, IEEE, 15 July 2011 (2011-07-15), pages 1310-1313, XP032031710, DOI: 10.1109/MACE.2011.5987183 ISBN: 978-1-4244-9436-1
• HAIDER S ET AL: "Magneto-acousto-electrical tomography: a potential method for imaging current density and electrical impedance; MAET: a potential method for imaging current density and electrical impedance", PHYSIOLOGICAL MEASUREMENT, INSTITUTE OF PHYSICS PUBLISHING, BRISTOL, GB, vol. 29, no. 6, 1 June 2008 (2008-06-01), pages S41-S50, XP020137116, ISSN: 0967-3334

## Description

### Field of the Invention

The present invention is related to medical imaging, in particular to electrical impedance imaging.

### State of the Art

Imaging electrical conductivity of biological tissues is one of the major research areas in the field of medical imaging. To image the conductivity of body tissues researchers have proposed different approaches. The earliest and generally accepted title for this relatively new imaging modality is Electrical Impedance Tomography (EIT) which uses surface electrodes to inject current and measures voltages from the body surface. Magnetic Resonance Electrical Impedance Imaging Tomography (MREIT), Magnetic Induction Tomography (MIT), Magneto Acoustic Tomography (MAT), Magneto-Acousto-Electrical Tomography (MAET) and Magneto Acoustic Tomography with Magnetic Induction (MAT-MI) are approaches proposed for the same purpose, but these methods use different means for applying currents and measurements.

In EIT, there are two major approaches: Applied Current Electrical Impedance Tomography (ACEIT) [1-3] and Induced Current Electrical Impedance Tomography (ICEIT) [4, 5]. In ACEIT electrodes are placed on the surface of a body. Electrical current is injected between a pair of electrodes and the resultant voltage is recorded between two surface electrodes [1]. Current drive and voltage measurement electrodes are changed to obtain an independent set of data for image reconstruction.

In MIT [6-9], currents are induced in the conductive body by time-varying magnetic fields using transmitter coils. The secondary magnetic fields due to existence of the conductive body are measured using receiver coils. The number of independent measurements is increased by changing the location of the transmitter coil.

In addition to these early attempts performed electromagnetically, recently, novel techniques are proposed that integrates electromagnetism with acoustics. MAT [10-12] is based on the idea that a force on the current pathway is generated by application of magnetic field to a liquid or tissue-like media in which electric currents are carried. This force is known as the Lorentz force. When the applied magnetic field is alternating, the generated force on the internal currents is also alternating. This time-varying force in the medium generates acoustic wave fronts which propagate away from the current site. These vibrations are detected at the object surface using ultrasonic transducers.

In MAET [13-19] technique the combination of electrical impedance tomography and ultrasound imaging is performed. In the existence of static magnetic field an ultrasonic pulse is propagated into the object, then an electrical current is induced and the surface voltage is measured through the electrodes. The electrical impedance of the object is reconstructed by using this voltage. Furthermore, an electrical pulse is propagated into the object and an ultrasound signal is detected with ultrasonic transducers.

In MAT-MI [20-23] technique, two magnetic fields are applied to the body: one static and one time-varying. Due to time-varying magnetic fields, eddy currents are generated. Eddy currents and applied static field result in vibrations (due to Lorentz force), and ultrasound waves are emitted. Ultrasonic transducers on the body surface are used as receivers. High resolution images of the conductivity distribution are reconstructed using this approach. The only difference between MAT-MI and MAT is that MAT uses electrical excitation under only static magnetic field. Since these approaches use ultrasound as the detected signal, the resultant conductivity images have high resolution.

Documents WO98/00732 constitutes further prior art disclosure of relevance in the context of the present invention.

### Aims of the Invention

The aims of the invention are:

- Obtaining images of conductivity and permittivity distributions of human body.
- Obtaining multi-frequency images.
- Obtaining a high resolution imaging system coupling static magnetic field and ultrasound,
- Obtaining a non-invasive imaging system for patients,
- Obtaining an imaging system that operates within safety limits.

### Brief Description of the Invention

In this invention, an approach is proposed to image the electrical impedance (conductivity and permittivity) properties of biological tissues. This approach is based on electrical current induction using ultrasound together with an applied static magnetic field. Acoustic vibrations are generated via piezoelectric transducers located on the surface of a biological body. A linear phased array piezoelectric transducer is used to form pressure distribution in human body/tissue. In the existence of a static magnetic field, the resultant (velocity) current density is sensed by a receiver coil encircling the tissue or placed near the tissue and used for reconstructing the conductivity and permittivity distribution.

**Description of the Figures**

The Figures and the related descriptions for the invention, multi frequency electrical impedance imaging system using Lorentz fields, described with this document are given below:

Figure-1: Geometry of the proposed approach

Figure-2: Proposed Electrical Impedance Imaging system

Figure-3: Detailed block diagram of the system for the proposed electrical impedance imaging method

Figure-4: X-coil configuration

Figure-5: Y-coil configuration

Figure-6: A single inhomogeneity (square domain of conductivity 0.8221 S/m) located at the center of the body (at a distance of 2.5 cm from the transducers) (The transducer is placed on the top side of the conductive body.)

Figure-7: A single inhomogeneity (square domain of conductivity 0.8221 S/m) located at the center of the body (at a distance of 2.5 cm from the transducers) (The transducer is placed on the left side of the conductive body.

Figure-8: Five identical inhomogeneities (each square domain of conductivity 0.8221 S/m) located symmetrically in the imaging domain. One is at the center of the body; others are at 1 cm distance from the body surfaces (The transducer is placed on the top side of the conductive body.)

Figure-9: Five identical inhomogeneities (each square domain of conductivity 0.8221 S/m) located symmetrically in the imaging domain. One is at the center of the body; others are at 1 cm distance from the body surfaces (The transducer is placed on the left side of the conductive body.)

Figure-10: Applied voltage (V) to each piezoelectric crystal.

Figure-11: The reconstructed image of Model 1 when the SNR is 40 dB (a) and 80 dB (b). Data is acquired using two transducer positions.

Figure-12: The reconstructed image of Model 2 when the SNR is 40 dB (a) and 80 dB (b). Data is acquired using two transducer positions.

**Description of the Components/Parts of the Invention**

The parts and components in the figures of the invention, described in this document are numbered for better understanding. The numerals referred to in the following description correspond to the following:

1- Ultrasonic transducer (single element and linear phased array)
2- Object to be imaged
3- Receiver coil (x-coil and y-coil)
4- Block diagram of the system for the proposed electrical impedance imaging method
5- Control unit of the imaging system
6- Signal Generator
7- Power Amplifier
8- Static Magnetic Field Generator
9- (Pre-)Amplifier + Lock-in Amplifier
10- Filter
11- (Post-)Amplifier

12- Data Acquisition Card (DAC)
13- Memory
14-Analog to Digital Converter (ADC)
15-Current path
16-Inhomogeneity (Tumor)

**Detailed Description of the Invention**

The steps of operation for this invention are described in this document basically comprising;

- Applying a uniform static magnetic flux density with the static magnetic field generator (8),
- Exciting the ultrasonic transducer (1) sinusoidally at its resonance frequency, $f_r$, with signal generator (6) and power amplifier (7),
- Steering the linear phased array transducer (1) with different angles,
- (A pressure wave is generated inside the object (2) and (velocity) current density is induced due to pressure and static magnetic field inside the object (2))
- Measuring the magnetic fields of the induced current by receiver coils (3),
- Amplify the measured signal by pre-amplifier and Lock-in Amplifier (9),
- Filtering the amplified signal by filter (10),
- Amplifying the filtered signal by post-amplifier (11),
- Transmitting the filtered signal to the DAC (12),
- Converting the analog signal to the digital signal by ADC (14),
- Controlling the digital signal with control unit (5),
- Reconstructing the conductivity and permittivity images with a computer program.

The theoretical background of the proposed approach is given below in details.

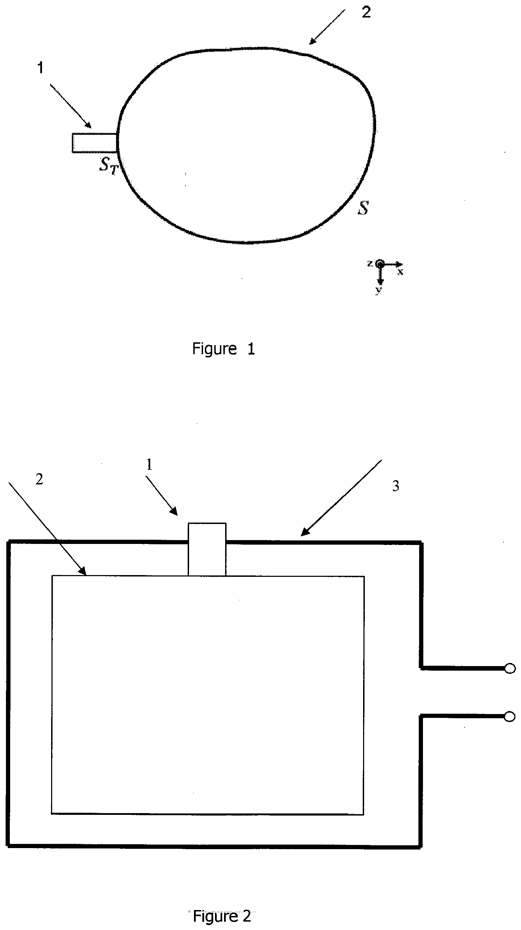

The proposed imaging modality is a *multiphysics* problem. To develop a numerical model the electromagnetic and acoustics fields must be solved simultaneously (Figure 1). First the basic field equations governing the behavior of time-varying electromagnetic and acoustic fields are given. Secondly, the general formulation of the partial differential equations for the scalar and magnetic vector potentials are presented in the electromagnetic part of this invention. In the acoustic part, the formulation of the partial differential equation for the acoustic pressure is presented. Thereafter, relation of the measurements to the existing (coupling) electromagnetic and acoustic waves is described.

### *1.1. Basic Electromagnetic Field Equations*

The following set of Maxwell's equations governs the behavior of time-varying electromagnetic fields in a linear, non-magnetic, isotropic conductive body [24]:

$$\nabla \times \vec{E} = -\frac{\partial \vec{B}}{\partial t} \qquad (E.1)$$

$$\nabla \times \vec{B} = \mu_0 \vec{J} + \mu_0 \frac{\partial \vec{D}}{\partial t} \qquad (E.2)$$

$$\nabla \cdot \vec{D} = \rho \qquad (E.3)$$

$$\nabla \cdot \vec{B} = 0 \qquad (E.4)$$

For the solution of these fields we need the continuity condition

$$\nabla \cdot \vec{J} = -\frac{\partial \rho}{\partial t} \qquad (E.5)$$

and the constitutive relations:

$$\vec{D} = \epsilon \vec{E} \qquad\qquad (E.6)$$

$$\vec{J} = \sigma \vec{E} + \sigma\left(\vec{v} \times \vec{B}\right) \qquad\qquad (E.7)$$

where $\vec{v}$ is the velocity of the conductor and $\vec{v} \times \vec{B}$ is the *Lorentz field* The second term on the right hand side of this equation is known as the *velocity* current density.

Since the divergence of $\vec{B}$ is zero, it is possible to introduce a magnetic vector potential $\vec{A}$ as

$$\vec{B} = \nabla \times \vec{A} \qquad\qquad (E.8)$$

Consequently, $\vec{E}$ can be expressed in terms of the magnetic vector potential $\vec{A}$ and gradient of a scalar potential $\varphi$ as

$$\vec{E} = -\nabla\varphi - \frac{\partial \vec{A}}{\partial t} \qquad\qquad (E.9)$$

In three-dimensional (3D) problems, the electric and magnetic fields are usually calculated using a $A$ - $\varphi$ formulation which results in two coupled equations in terms of $\vec{A}$ and $\varphi$. To obtain the first equation, Equation (E.2) is rewritten in terms of the magnetic vector potential $\vec{A}$ as follows:

$$\nabla \times \mu_0^{-1}\left(\nabla \times \vec{A}\right) = \vec{J} + \frac{\partial \vec{D}}{\partial t} \qquad\qquad (E.10)$$

The terms $\vec{J}$ and $\vec{D}$ on right hand side can be expressed using equations (E.6) and (E.7) yielding:

$$\nabla \times \mu_0^{-1}\left(\nabla \times \vec{A}\right) = \sigma\left(\vec{E} + \vec{v} \times \vec{B}\right) + \frac{\partial}{\partial t}\epsilon\vec{E} \qquad\qquad (E.11)$$

Reorganizing the right hand side we obtain,

$$\nabla \times \mu_0^{-1}\left(\nabla \times \vec{A}\right) = (\sigma + \epsilon\frac{\partial}{\partial t})\vec{E} + \sigma\overrightarrow{(\vec{v} \times \vec{B})} \qquad\qquad (E.12)$$

By replacing $\vec{B}$ and $\vec{E}$ using the expressions in (E.8) and (E.9) we obtain,

$$\nabla \times \mu_0^{-1}\left(\nabla \times \vec{A}\right) + \left(\sigma + \epsilon\frac{\partial}{\partial t}\right)\left(\nabla\varphi + \frac{\partial \vec{A}}{\partial t}\right) - \sigma(\vec{v} \times \nabla \times \vec{A}) = 0 \qquad (E.13)$$

A second equation relating $\vec{A}$ and $\varphi$ can be obtained using the continuity equation (Equation (E.5)),

$$\nabla \cdot \vec{J} = \nabla \cdot \sigma\left(\vec{E} + \vec{v} \times \vec{B}\right) = -\frac{\partial}{\partial t}\nabla \cdot (\epsilon\vec{E}) \qquad\qquad (E.14)$$

$$\nabla \cdot \sigma\left(\vec{E} + \vec{v} \times \vec{B}\right) + \frac{\partial}{\partial t}\nabla \cdot \left(\epsilon\vec{E}\right) = 0 \qquad\qquad (E.15)$$

$$\nabla \cdot \left[\left(\sigma + \frac{\partial}{\partial t}\epsilon\right)\vec{E} + \sigma(\vec{v} \times \vec{B})\right] = 0 \qquad (E.16)$$

Once again, using the expressions in equations (E.8) and (E.9), we obtain

$$\nabla \cdot \left[\left(\sigma + \frac{\partial}{\partial t}\epsilon\right)\left(\nabla\varphi + \frac{\partial \vec{A}}{\partial t}\right) - \sigma(\vec{v} \times \nabla \times \vec{A})\right] = 0 \qquad (E.17)$$

Equations (E.13) and (E.17) represent the general form of the system of equations that is used to calculate $\vec{A}$ and $\varphi$ for arbitrary excitations. For sinusoidal excitations ($e^{j\omega t}$ is assumed), these two equations can be written using phasor notation as follows:

$$\nabla \times \mu_0^{-1}\left(\nabla \times \vec{A}\right) + (\sigma + j\omega\epsilon)\left(\nabla\varphi + j\omega\vec{A}\right) - \sigma\left(\vec{v} \times \nabla \times \vec{A}\right) = 0 \qquad (E.18)$$

$$\nabla \cdot \left[(\sigma + j\omega\epsilon)\left(\nabla\varphi + j\omega\vec{A}\right) - \sigma\left(\vec{v} \times \nabla \times \vec{A}\right)\right] = 0 \qquad (E.19)$$

In the proposed system, the conductive body (2) is source-free, i.e., the low-frequency biological sources and corresponding potentials are of no concern. The potentials $A$ and $\varphi$ can be solved once the boundary conditions are set due to external sources. In the proposed approach, neither a time-varying magnetic field is applied using external coils nor a current or potential is applied using, for example, surface electrodes. The origin of the electric and magnetic potentials is the Lorentz fields and currents generated by the combination of a uniform static magnetic field $\vec{B}_0$ (say, in z direction) and a propagating acoustic field. The latter is generated by an ultrasound transducer (1) attached on the surface of the body (2) as shown in Figure 1. Equations (E.18) and (E.19) together with appropriate boundary conditions can be used to calculate the electric field components for general conditions, i.e., general material properties and excitation frequencies.

### 1.2. Basic Acoustic Field Equations

To provide simplicity, a lossless, source-free medium is assumed and, initially, a one-dimensional (1-D) derivation is presented. In the static case, the body pressure is constant and denoted by $p_0$. The mass density of the body is assumed position dependent and represented by $\rho_0(x)$. In the presence of a propagating pressure wave, the total pressure $p_T$ is position and time-varying and is given as

$$p_T(x, t) = p_0 + p(x, t) \qquad (E.20)$$

In such a case, the total mass density can be expressed as

$$\rho_T(x, t) = \rho_0 + \rho(x, t) \qquad (E.21)$$

A propagating ultrasound also result in displacements in the small elements of the body and an associated 'particle velocity', $\vec{v}(x, t)$. The particle displacement and its derivatives are small when $|p/p_0| \ll 1$ and $|\rho/\rho_0| \ll 1$. This results in a linearization procedure, and the 'equation of motion' is expressed as [25] :

$$-\partial p/\partial x = \rho_0 \; \partial v/\partial t \qquad (E.22)$$

This shows that a particle accelerates in the opposite direction of the pressure gradient. The 'continuity equation' for mass conservation is written as

$$- \frac{\partial \rho_T}{\partial t} = \frac{\partial(\rho_T v)}{\partial x} \qquad (E.23)$$

which reduces to the following after linearization (under the above given conditions):

$$- \frac{\partial \rho}{\partial t} = \rho_0 \frac{\partial v}{\partial x} \qquad (E.24)$$

To obtain a differential equation relating $p$ and $\rho$, the particle velocity term in Equations (E.22) and (E.24) must be eliminated. This can be achieved by employing a 'constitutive equation' between them. If the change in mass density $\rho$ is some function of changes in pressure $p$ only, then a 'linearized constitutive equation' can be written as

$$\rho = [\partial \rho / \partial p]_{p=0} \ p = \rho_0 \beta_0 \ p \qquad (E.25)$$

where $\beta_0$ is the compressibility (reciprocal of the bulk modulus or elastic constant) defined as

$$\beta_0 = - \frac{1}{V} \frac{\partial V}{\partial p}$$

Three dimensional wave equation for pressure is derived by combining Equations (E.23), (E.24) and (E.25) as:

$$\frac{1}{\rho_0 c_s^2} \frac{\partial^2 p}{\partial t^2} = \nabla \cdot \left( \frac{1}{\rho_0} \ \nabla p \right) \qquad (E.26)$$

where $c_s^2 = (\rho_0 \beta_0)^{-1}$ represents the speed of pressure waves.

The equation of motion (Equation (E.22)) used in the preceding derivation is true as long as there are no other force terms. We may drop the terms related to gravitational force, whereas presence of current density $\vec{J}$ and magnetic flux density $\vec{B}$ in the body results in Lorentz force (per unit volume) $\vec{q} = \vec{J} \times \vec{B}$ in addition to the mechanical forces in the body. Consequently, in the presence of a magnetic flux density and charged particles in the body (2), equation of motion can be modified as follows:

$$q_x - \partial p / \partial x = \rho_0 \ \partial v / \partial t \qquad (E.27)$$

where $q_x$ denotes the x-component of this interaction. Note that, movement of charged particles results in a current density $\vec{J} = \sigma \vec{E} + \sigma(\vec{v} \times \vec{B})$, in turn, current density influences the motion. In such a case, the wave equation should be modified taking into account the effects of Lorentz forces, yielding

$$\frac{1}{\rho_0 c_s^2} \frac{\partial^2 p}{\partial t^2} = \nabla \cdot \left[ \frac{1}{\rho_0} \ (\nabla p - \vec{q}) \right] \qquad (E.28)$$

Since the medium is source-free, the pressure distribution is determined due to boundary conditions as dictated by an ultrasonic transducer (1) in contact with the medium. In this study, the boundary conditions are assumed as follows:

$$\frac{1}{\rho_0} \ (\nabla p - \vec{q}) \cdot \vec{n} = a_n \ \ on \ S_T \qquad (E.29)$$

where $S_T$ denotes the body(2) surface which is in contact with the transducer, The term $a_n$ in Equation (E.29) represents the *local acceleration* produced by the transducer (1) (derivation of this term is discussed in the next section). The remaining part of the surface is denoted by $S$. The second boundary condition is obtained using the continuity relation for acceleration:

$$\frac{\partial \vec{v}_1}{\partial t} \cdot \vec{n} = \frac{\partial \vec{v}_2}{\partial t} \cdot \vec{n}$$

where $\vec{v}_1$ and $\vec{v}_2$ denote the particle velocities in region 1 and region 2 of an interface, respectively. This expression can also be written as

$$\frac{1}{\rho_{01}} (\nabla p - \vec{q})_1 \cdot \vec{n} = \frac{1}{\rho_{02}} (\nabla p - \vec{q})_2 \cdot \vec{n} \qquad on \ S \qquad (E.30)$$

### 1.3. Piezoelectric Medium:

Piezoelectric materials, which normally have neutral molecules, respond to an applied electric field by changing their mechanical dimensions.

Converse is also true; when a piezoelectric material is strained an electric field occurs due to small electric dipoles generated inside the material. This is due to their asymmetric atomic lattice. The field equations of piezoelectric materials couples the equations of elasticity and electricity by piezoelectric *constitutive relations* as explained below.

Force per unit area applied to a body is called *stress* and, in one-dimensional (1D) case, it is denoted by *T*. The fractional extension of the body is called *strain* and is denoted by *S*. For small stresses applied to a 1D system, the relation between stress and strain is given by the Hooke's law:

$$T = cS$$

where $c = 1/\beta_0$ is the *elastic constant* of the material. In a piezoelectric material, however, the piezoelectric constitutive relation is as given below:

$$T = c^E S - eE \qquad (E.31)$$

The additional stress term on the right hand side is due to the presence of the electric field *E*. The parameter *e* is called the *piezoelectric stress constant,* and $c^E$ is the *elastic constant in the presence of constant or zero E field.*
In the presence of an electric field *E*, the electrical displacement D depends on the strain as well as the electric field:

$$D = eS + \epsilon^S E \qquad (E.32)$$

where $\epsilon^S$ is the *permittivity* with zero or constant strain.
If the material is not piezoelectric, the 'equation of motion', say in z-direction, is given by:

$$\frac{\partial T}{\partial z} = \rho_0 \frac{\partial v}{\partial t} \qquad (E.33)$$

or

$$c^E \frac{\partial^2 u}{\partial z^2} = \rho_0 \frac{\partial^2 u}{\partial t^2} \qquad (E.34)$$

which is another form of Equation (E.26) in terms of displacement u when the medium has uniform material properties. The electrical behavior of the medium is governed by the following equation for the z component of the electrical displacement vector $D_z$,

$$\frac{\partial D_z}{\partial z} = 0 \qquad\qquad (E.35)$$

implying that $D_z$ is constant and there are no free charges in the medium. The electric field can be written in terms of the derivative of a scalar potential as follows:

$$E = -\frac{\partial \varphi}{\partial x}$$

Consequently, Equation (E.31) can be rewritten as:

$$-\epsilon^s \frac{\partial^2 \varphi}{\partial x^2} = 0 \qquad\qquad (E.36)$$

If the material is not piezoelectric, Equations (E.31) and (E.32) are isolated. In a piezoelectric material, however, the elasticity and electricity equations are coupled due to piezoelectric constitutive relations (E.31) and (E.32). In such a case, one obtains the following equations of piezoelectricity in 1D:

$$c^E \frac{\partial^2 u}{\partial z^2} + e \frac{\partial^2 \varphi}{\partial z^2} = \rho_0 \frac{\partial^2 u}{\partial t^2} \qquad\qquad (E.37)$$

$$e \frac{\partial^2 u}{\partial z^2} - \epsilon^s \frac{\partial^2 \varphi}{\partial x^2} = 0 \qquad\qquad (E.38)$$

In a 3D problem, three equations are derived in the elasticity part for the three displacement components. Together with the electric field equation, four equations are obtained for four unknowns. Depending on the material properties, different forms of these equations can be found [26].

### 1.4. Relation of measurements to the conductivity distribution: Magnetic Field Measurements (Proposed Approach)

#### Lead-Field analysis:

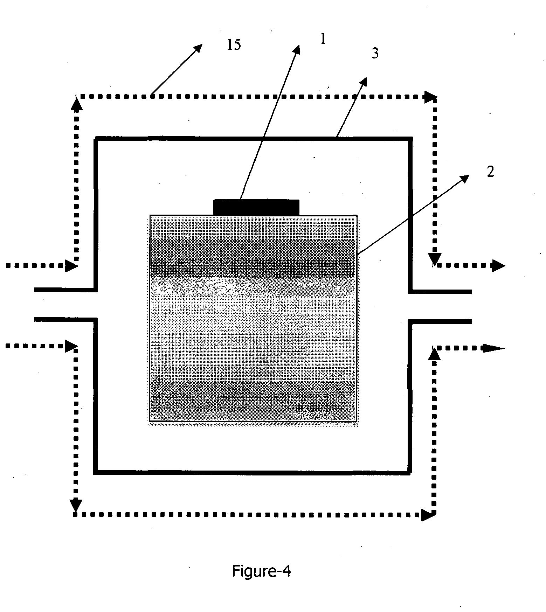

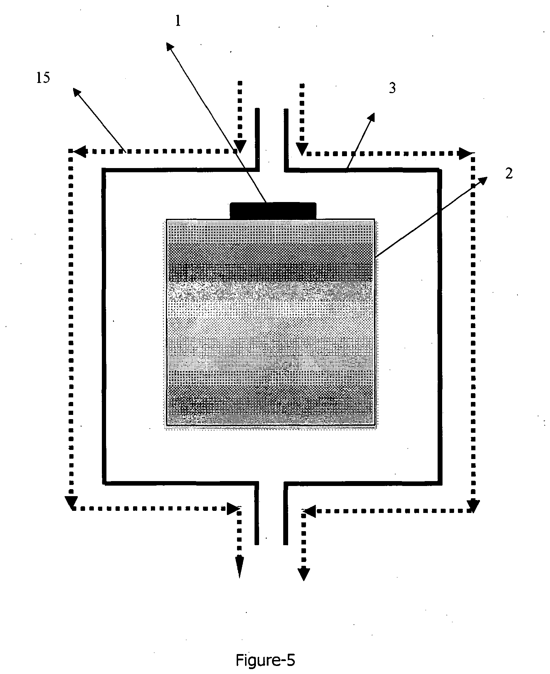

The measured data in the proposed approach are the voltages picked up from the receiver coil (3) (Figure 4 and 5). The relation between the receiver coil (3) voltage and magnetic flux density is given by the Faraday's law of induction:

$$v_{ab}(t) = -\int_{Coil} \frac{\partial \vec{B}}{\partial t} \cdot d\vec{S} \qquad\qquad (E.39)$$

where $d\vec{S}$ is the differential surface element in the surface enclosed by the receiver coil (3). Since $\vec{B}(t)$ is related to the conductivity and permittivity distributions in the body, the received voltage is a function of the conductivity and permittivity distributions. The above equation, however, does not show this relation explicitly. To obtain such a relation, in this study, an approach that is frequently used in formulating the forward problem of magnetoencephalography (MEG) is used. This approach is based on the *reciprocity theorem* [27]. In short, this theorem states that the location of the detector and source can be changed without affecting the detected signal amplitude. Using this theorem, the detected signal is expressed in terms of volume integral of the source (dipole) distribution. The sensitivity of the measurement to a specific dipole is determined by the scalar product of a *lead-field vector* (the electric field generated by a reciprocal unit current in the detector coil) with the selected dipole. Thus, once the lead field vector is solved for specific detector geometry, the detector voltage for an arbitrary dipole source can be easily calculated by integrating over the source domain.

Similar approach can be applied if the lead field vector for the proposed imaging modality can be identified. In the following sections, the lead field vector will be found for electromagnetic fields with 1) harmonic time dependence, and 2) general time dependence.

*Harmonic time dependence:*

A straightforward extension of this imaging modality is to excite the ultrasonic transducer (1) with its resonance frequency continuously. The pick-up voltage at the receiver coil (3) is then the steady state response (a single measurement) obtained for a specific transducer/receiver coil (3) configuration. To increase the number of measurements, the transducer and receiver coil (3) configurations should be changed. Though the measurement strategy is somehow difficult for continuous excitation, the analysis for the lead field vector analysis is relatively simple.

In the direct problem, $\vec{E}_1$ and $\vec{H}_1$ represent the electric field and magnetic field in the body (2) when currents are ultrasonically induced in the body (2). Assuming $e^{j\omega t}$ time dependence and adopting the boldface phasor notation, the following equations are valid,

$$\nabla \times \vec{E}_1 = -j\omega\mu_0\vec{H}_1 \qquad (E.40)$$

$$\nabla \times \vec{H}_1 = \sigma\vec{E}_1 + \vec{J}_1 \qquad (E.41)$$

where $\vec{J}_1 = \sigma(v \times \vec{B}_0)$ denotes the velocity current due to ultrasonic excitation under a static magnetic field.

In the reciprocal problem there are no current sources in the body (2). The electric field $\vec{E}_2$ and the magnetic field intensity $\vec{H}_2$ are generated inside the body (2) due to a reciprocal current density $\vec{J}_2$ (or $\vec{J}_R$) inside the receiver coil (3) of the direct problem. In the reciprocal problem the following equation must be satisfied:

$$\nabla \times \vec{E}_2 = -j\omega\mu_0\vec{H}_2 \qquad (E.42)$$

$$\nabla \times \vec{H}_2 = \sigma\vec{E}_2 + \vec{J}_2 \qquad (E.43)$$

Using Equations (E.40) and (E.43) one obtains the following equation:

$$\vec{H}_2 \cdot \nabla \times \vec{E}_1 - \vec{E}_1 \cdot \nabla \times \vec{H}_2 = -j\omega\mu_0\vec{H}_1 \cdot \vec{H}_2 - \sigma\vec{E}_2 \cdot \vec{E}_1 - \vec{J}_2 \cdot \vec{E}_1 \qquad (E.44)$$

Similarly, using Equations (E.41) and (E.42) we obtain

$$\vec{H}_1 \cdot \nabla \times \vec{E}_2 - \vec{E}_2 \cdot \nabla \times \vec{H}_1 = -j\omega\mu_0\vec{H}_1 \cdot \vec{H}_2 - \sigma\vec{E}_2 \cdot \vec{E}_1 - \vec{J}_1 \cdot \vec{E}_2 \qquad (E.45)$$

Using the vector identity $\nabla\times(\vec{A}\times\vec{B})=\vec{B}\cdot(\nabla\times\vec{A})-\vec{A}\cdot(\nabla\times\vec{B})$, the left hand sides of (E.44) and (E.45) are simplified and equations are put in the following form:

$$\nabla \cdot (\vec{E}_1 \times \vec{H}_2) = -j\omega\mu_0\vec{H}_1 \cdot \vec{H}_2 - \sigma\vec{E}_2 \cdot \vec{E}_1 - \vec{J}_2 \cdot \vec{E}_1 \qquad (E.46)$$

$$\nabla \cdot (\vec{E}_2 \times \vec{H}_1) = -j\omega\mu_0\vec{H}_1 \cdot \vec{H}_2 - \sigma\vec{E}_2 \cdot \vec{E}_1 - \vec{J}_1 \cdot \vec{E}_2 \qquad (E.47)$$

By subtracting Equation (E.47) from Equation (E.46),

$$\nabla \cdot (\vec{E}_1 \times \vec{H}_2) - \nabla \cdot (\vec{E}_2 \times \vec{H}_1) = \vec{J}_1 \cdot \vec{E}_2 - \vec{J}_2 \cdot \vec{E}_1 \qquad (E.48)$$

Taking the volume integral in all universe (in volume $V_\infty$ bounded by $S_\infty$) we obtain

$$\int_{V_\infty} \nabla \cdot (\vec{E}_1 \times \vec{H}_2 - \vec{E}_2 \times \vec{H}_1) dV = \int_{V_\infty} (\vec{J}_1 \cdot \vec{E}_2 - \vec{J}_2 \cdot \vec{E}_1) dV \qquad (E.49)$$

By applying the divergence theorem to the left hand side it becomes a surface integral,

$$\int_{V_\infty} \nabla \cdot \left(\vec{E}_1 \times \vec{H}_2 - \vec{E}_2 \times \vec{H}_1\right) dV = \oint_{S_\infty} \vec{E}_1 \times \vec{H}_2 - \vec{E}_2 \times \vec{H}_1 dS \qquad \text{(E.50)}$$

Since the electric and magnetic field intensities vanish at infinity, we obtain the well-known reciprocity relation:

$$\int_{V_\infty} \left(\vec{J}_1 \cdot \vec{E}_2 - \vec{J}_2 \cdot \vec{E}_1\right) dV \qquad \text{(E.51)}$$

Since the current density $\vec{J}_1$ in the direct problem is nonzero in the conducting body volume ($V_{body}$) and the current density $\vec{J}_2$ in the reciprocal problem is nonzero in the receiver coil volume ($V_{coil}$), Equation (E.51) reduces to the following:

$$\int_{V_{body}} \vec{J}_1 \cdot \vec{E}_2 \, dV = \int_{V_{coil}} \vec{J}_2 \cdot \vec{E}_1 \, dV \qquad \text{(E.52)}$$

Recognizing that $\vec{J}_2 dV = I_R \vec{dl}$, the right hand side becomes

$$\int_{V_{body}} \vec{J}_1 \cdot \vec{E}_2 \, dV = I_R \int_{V_{coil}} \vec{E}_1 \cdot \vec{dl} \qquad \text{(E.53)}$$

The integral on the right hand side is the pick-up voltage $V_{ab}$ due to ultrasonically induced harmonic currents in the body (2). Consequently, $V_{ab}$ is expressed as follows:

$$V_{ab} = \int_{V_{body}} \vec{J}_1 \cdot \left(\vec{E}_R / I_R\right) dV \qquad \text{(E.54)}$$

where $\vec{E}_2$ is replaced by $\vec{E}_R$. The term in the parenthesis is the electric field in the reciprocal problem when unit current is applied to the receiver coil (3) and it is called as the *lead field vector* ($\vec{L}_M$) for the forward problem of the proposed imaging modality. The subscript $M$ in the lead field vector shows that it is the lead field vector for the magnetic field measurements. Note that the lead-field vector itself is a function of body conductivity.

To show the relation between the pick-up voltage and conductivity in the medium, the term $\vec{J}_1$ is replaced by $\sigma(v \times \vec{B}_0)$ and the final form is obtained:

$$V_{ab} = \int_{V_{body}} \sigma\left(v \times \vec{B}_0\right) \cdot \vec{L}_M(\sigma) dV \qquad \text{(E.55)}$$

Note that the pick-up voltage is linearly proportional to the conductivity distribution and the weight of each conductive element is determined by the dot product of the Lorentz field and reciprocal field on that element.

### General time dependence:

When general time dependence is assumed for the particle velocity $\vec{v}(t)$, the reciprocity relation given in Equation (E.51) does not hold. Instead, the following relation is valid [46]:

$$\int_{-\infty}^{\infty} dt \int_{V_{body}} dV \vec{J}_1(\vec{r}, \tau - t) \cdot \vec{E}_2(\vec{r}, t) = \int_{-\infty}^{\infty} dt \int_{V_{coil}} dV \vec{J}_2(\vec{r}, t) \cdot \vec{E}_1(\vec{r}, \tau - t) \qquad \text{(E.56)}$$

In this expression, $\vec{E}_1$ is the electric field in the direct problem due to velocity current $\vec{J}_1 = \sigma(\vec{v}(t) \times \vec{B})$ and $\vec{E}_2$ is the electric field in the reciprocal problem due to reciprocal current $\vec{J}_2 = \vec{J}_R$ in the receiver coil. Here $\vec{r}$ represents the three-dimensional position vector.

For the receiver coil, we may write

$$\int_{V_{coil}} dV \vec{J}_R(t) = \int_{Coil} d\vec{l} \, I_R(t)$$

Then the right hand side of Equation (E.56) becomes,

$$\int_{-\infty}^{\infty} dt \, I_R(t) \int_{Coil} E_1(\tau - t) \cdot d\vec{l} = \int_{-\infty}^{\infty} dt \, I_R(t) V_{ab}(\tau - t) = I_R * V_{ab} \qquad \text{(E.57)}$$

For the left hand side of Equation (E.56), we recognize that the electric field $\vec{E}_R(\vec{r},t)$ in the reciprocal problem can be written as the product of a position dependent function and energizing reciprocal current $I_R(t)$:

$$\vec{E}_R(\vec{r},t) = \vec{E}_R^0(\vec{r}) I_R(t) \qquad \text{(E.58)}$$

where $\vec{E}_R^0(\vec{r})$ is the reciprocal electric field normalized with unit current in the receiver coil (3). Since the body (2) is assumed resistive, Equation (E.58) should be valid. Consequently, we obtain

$$\int_{-\infty}^{\infty} dt \int_{V_{body}} dV \vec{J}_1(\vec{r}, \tau - t) \cdot \vec{E}_R(\vec{r},t) = \int_{-\infty}^{\infty} dt \, I_R(t) \int_{V_{body}} dV \vec{J}_1(\vec{r}, \tau - t) \cdot \vec{E}_R^0(\vec{r}) \qquad \text{(E.59)}$$

Comparison of Equations (E.57) and (E.59) shows that

$$V_{ab}(\tau - t) = \int_{V_{body}} dV \vec{J}_1(\vec{r}, \tau - t) \cdot \vec{E}_R^0(\vec{r}) \qquad \text{(E.60)}$$

A final form of this equation can be obtained by 1) change of variables for the time variation, 2) dropping the position vector $\vec{r}$, 3) expressing $\vec{J}_1$ in terms of particle velocity, and 4) setting $\vec{E}_R^0 = \vec{E}_R^0(\sigma)$.

$$V_{ab}(t) = \int_{V_{body}} dV \, \sigma (\vec{v}(t) \times \vec{B}_0) \cdot \vec{E}_R^0(\sigma) \qquad \text{(E.61)}$$

It is observed that for a given receiver coil (3) (and corresponding reciprocal field distribution), it is possible to acquire continuous data that gives information about the body conductivity along the propagation path of the particle velocity.

### 1.5. Receiver coil design considerations

When the ultrasonic transducer (1) position is fixed, the sensitivity in the measurements can be increased by optimizing the reciprocal field distribution. However, even for a fixed transducer (3) position ultrasonic beam steering approaches are applicable using an ultrasound array (1). Thus, in order to use a different receiver coil (3) for each beam direction, one should be able to acquire the best available data for any propagation direction. This can be achieved by using two coils for data acquisition, namely, x- and y-coils (3) which are sensitive to acoustic propagation in x- and y-directions, respectively. Figure 4 and Figure 5 shows two realizable coil (3) configurations encircling the body (1) for the development of x- and y-coils (3), respectively.

### 1.6. Simulation Examples

In this invention, the performance of the proposed imaging system (4) can be assessed by simulation studies. For simulation studies electrical and acoustic properties of homogeneous (2) and inhomogeneous (tumor) (16) bodies are defined. The conductive body (2) and tumor (16) are modeled as 5 cm x 5 cm and 5 mm x 5 mm in size, respectively. For conductive body (2) and tumor (16) electrical and acoustic properties of breast fat (2) and tumor (blood) (16) are represented. Electrical conductivities of breast fat (2) and tumor (blood) (16) are 0.0257 S/m and 0.8221 S/m, respectively at 1 MHz. The acoustic properties of breast fat (2) and tumor (16) are defined as: density of breast fat (2) is 980 kg/m$^3$, the speed of sound in it is 1520 m/s; density of tumor (16) is 1040 kg/m$^3$, the speed of sound in it is 1550 m/s.

To show the performance of the system, two models are prepared:

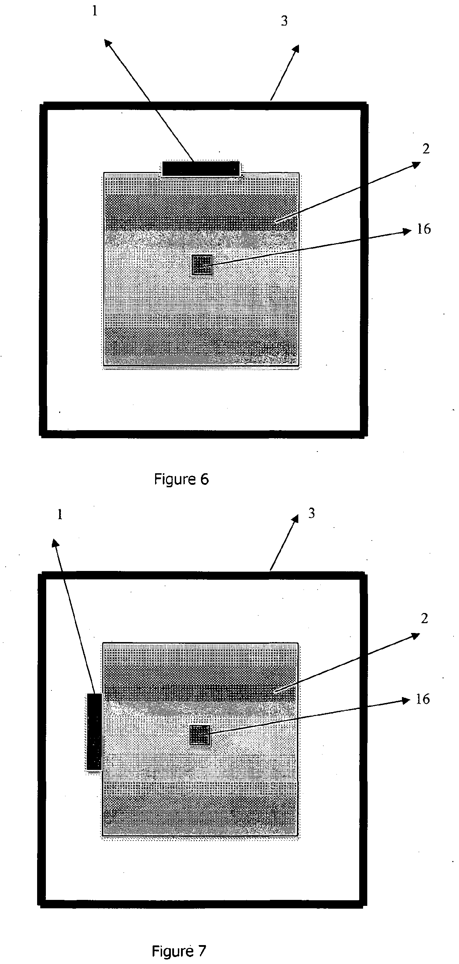

a) Model 1 : A single inhomogeneity (16) (square domain of conductivity 0.8221 S/m)) located at the center of the body (2) (at a distance of 2.5 cm from the transducers (1)) (Figure 6 and Figure 7)

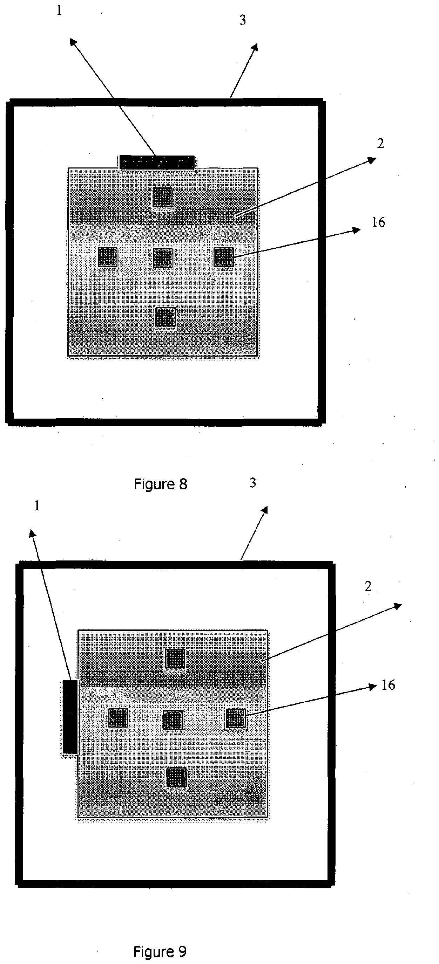

b) Model 2: Five identical inhomogeneities (16) (each square domain of conductivity 0.8221 S/m) located symmetrically in the imaging domain (2). One is at the center of the body (2); others are at 1 cm distance from the body (2) surfaces (Figure 8 and Figure 9)

A single transducer (1) (16-element linear phased array) is used for excitation. A sinusoidal voltage $V$ is applied to the surface of each element of linear phased array (1) for one period of the excitation frequency $f$, i.e.,

$$V(t) = A sin(2\pi ft) \quad (t<1/f) \quad\quad\quad (E.62)$$

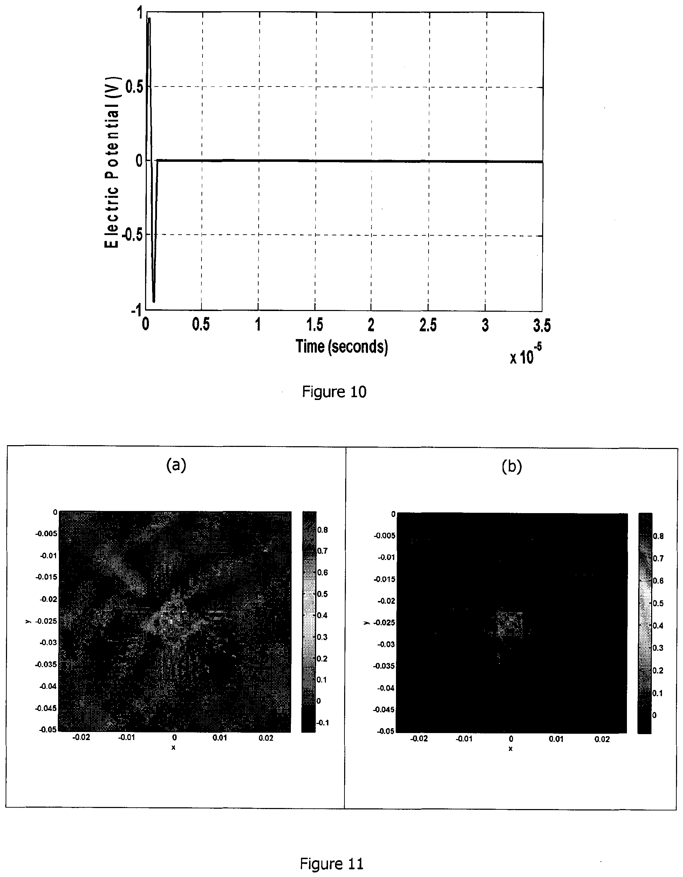

where the amplitude term A=1V and $f$=1 MHz (Figure 10).

The Truncated Singular Value Decomposition (SVD) method is used to reconstruct the images of the conductivity distribution. Initially, a single transducer (1) (16 -element linear phased array) is used for excitation as shown in Figure 10. It is excited for seven steering angles (-22.5°, -15°, -7.5°, 0°, 7.5°, 15°, 22.5°). To assess the reconstruction performance in the case of noisy data Gaussian noise is added to the measurements. To equalize the resolution in both directions, data is acquired for a second transducer (1) position (Figure 7). Images of the first model (Model 1) are reconstructed according to the Signal-to-Noise Ratio (SNR)s of 40 dB and 80 dB as shown in Figure 11 (a) and (b), respectively.

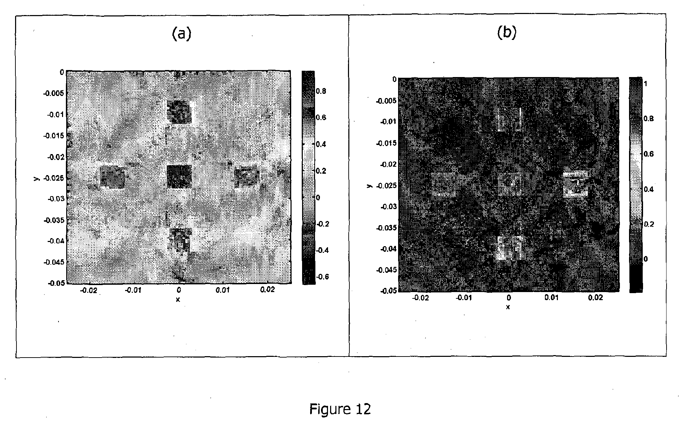

Similar procedure is implemented for Model 2. The reconstructed images according to SNRs of 40 dB and 80 dB are shown in Figure 12 (a) and (b), respectively.

## 2. REFERENCES

[1] D. C. Barber, and B. H. Brown, "APPLIED POTENTIAL TOMOGRAPHY," Journal of Physics E-Scientific Instruments, vol. 17, no. 9, pp. 723-733, 1984.

[2] K. Paulson, W. Lionheart, and M. Pidcock, "OPTIMAL EXPERIMENTS IN ELECTRICAL-IMPEDANCE TOMOGRAPHY," Ieee Transactions on Medical Imaging, vol. 12, no. 4, pp. 681-686, Dec, 1993.

[3] P. Metherall, D. C. Barber, R. H. Smallwood, and B. H. Brown, "Three-dimensional electrical impedance tomography," Nature, vol. 380, no. 6574, pp. 509-512, Apr, 1996.

[4] N. G. Gencer, "Electrical impedance tomography using induced currents," Electrical and Electronics Engineering, Middle East Technical University, 1993.

[5] N. G. Gencer, M. Kuzuoglu, and Y. Z. Ider, "ELECTRICAL-IMPEDANCE TOMOGRAPHY USING INDUCED CURRENTS," Ieee Transactions on Medical Imaging, vol. 13, no. 2, pp. 338-350, Jun, 1994.

[6] A.-Z. S, G. D, L. G, Y. Z. Z, P. A. J, and B. M. S, "A feasibility study of electromagnetic induction tomography." pp. 426-9.

[7] S. Al-Zeibak, D. Goss, G. Lyon, Z. Z. Yu, A. J. Peyton, and M. S. Beck, "A feasibility study of electromagnetic induction tomography." pp. 426-9.

[8] N. G. Gencer, and N. Tek, "Imaging Tissue Conductivity via Contactless Measuremens: A Feasibility Study," 6(3), http://journals.tubitak.gov.tr/elektrik/issues/elk-98-6-3/elk-6-3-2-98024.pdf 1998].

[9] H. Griffiths, "Magnetic induction tomography," Measurement Science & Technology, vol. 12, no. 8, pp. 1126-1131, Aug, 2001.

[10] B. C. Towe, and M. R. Islam, "A MAGNETOACOUSTIC METHOD FOR THE NONINVASIVE MEASUREMENT OF BIOELECTRIC CURRENTS," Ieee Transactions on Biomedical Engineering, vol. 35, no. 10, pp. 892-894, Oct, 1988.

[11] B. J. Roth, P. J. Basser, and J. P. Wikswo, "A THEORETICAL-MODEL FOR MAGNETOACOUSTIC IMAGING OF BIOELECTRIC CURRENTS," Ieee Transactions on Biomedical Engineering, vol. 41, no. 8, pp. 723-728, Aug, 1994.

[12] B. J. Roth, and J. P. Wikswo, "Comments on "Hall effect imaging"," Ieee Transactions on Biomedical Engineering, vol. 45, no. 10, pp. 1294-1295, Oct, 1998.

[13] H. Wen, J. Shah, and R. S. Balaban, "Hall effect imaging," Ieee Transactions on Biomedical Engineering, vol. 45, no. 1, pp. 119-124, Jan, 1998.

[14] H. Wen, and R. S. Balaban, *ULTRASOUND ARRAY AND ELECTRODE ARRAY FOR HALL EFFECT IMAGING,* WO WO 2000/019244 A1, D. O. H. The Government Of The United States Of America represented by The Secretary, S. Human, H. Wen and R. S. Balaban, 2000.

[15] H. Wen, *ULTRASOUND-HALL EFFECT IMAGING SYSTEM AND METHOD,* WO WO 1998/000732 A1, D. O. H. The Government Of The United States Of America represented by The Secretary, S. Human and H. Wen, 1998.

[16] H. Wen, "Volumetric Hall effect tomography - A feasibility study," Ultrasonic Imaging, vol. 21, no. 3, pp. 186-200, Jul, 1999.

[17] A. Montalibet, J. Jossinet, A. Matias, and D. Cathignol, "Electric current generated by ultrasonically induced Lorentz force in biological media," Medical & Biological Engineering & Computing, vol. 39, no. 1, pp. 15-20, Jan, 2001.

[18] Y. Xu, S. Haider, and A. Hrbek, "Magneto-acousto-electrical tomography: A new imaging modality for electrical impedance." pp. 292-295.

[19] S. Haider, A. Hrbek, and Y. Xu, "Magneto-acousto-electrical tomography: a potential method for imaging current density and electrical impedance," Physiological Measurement, vol. 29, no. 6, pp. S41-S50, Jun, 2008.

[20] Y. Xu, and B. He, "Magnetoacoustic tomography with magnetic induction (MAT-MI)," Physics in Medicine and Biology, vol. 50, no. 21, pp. 5175-5187, Nov, 2005.

[21] X. Li, Y. Xu, and B. He, "Magnetoacoustic tomography with magnetic induction for imaging electrical impedance of biological tissue," Journal of Applied Physics, vol. 99, no. 6, Mar, 2006.

[22] R. M. Xia, X. Li, B. He, and Ieee, Magnetoacoustic tomography of biological tissue with magnetic induction, 2007.

[23] K. Brinker, and B. J. Roth, "The effect of electrical anisotropy during magnetoacoustic tomography with magnetic induction," Ieee Transactions on Biomedical Engineering, vol. 55, no. 5, pp. 1637-1639, May, 2008.

[24] R. Plonsey, and R. Collin, Principles and Applications of Electromagnetic Fields, NewYork: Mc.Graw-Hill, 1961.

[25] M. Carley, Some Notes on Acoustics, 2001.

[26] T. G. Zielinski, "Fundamentals of multiphysics modelling of piezo-poro-elastic structures," Archives of Mechanics, vol. 62, no. 5, pp. 343-378, 2010.

[27] J. R. Mortarelli, "A GENERALIZATION OF THE GESELOWITZ RELATIONSHIP USEFUL IN IMPEDANCE PLETHYSMOGRAPHIC FIELD CALCULATIONS," Ieee Transactions on Biomedical Engineering, vol. 27, no. 11, pp. 665-667, 1980.

**Claims**

1. A method for producing images of the electrical properties of a specimen (2) comprising:

**a.** Generating via a static magnetic field generator (8) a static magnetic field inside the specimen (2)

**b.** in the presence of the static magnetic field, applying an acoustic wave to the specimen (2), by exciting an ultrasonic transducer (1) located on the surface of the specimen (2) with a sinusoidal voltage of a specific excitation frequency

**c.** detecting via a receiver coil (3) encirling the specimen or placed near the specimen (2) the Lorentz electrical field induced by the propagation of the acoustic wave inside de specimen

**d.** generating images showing the conductivity and permittivity distributions of the specimen (2) based on the detected Lorentz electrical field.

2. The method according to claim 1, wherein the ultrasonic transducer (I) is a single element or linear phased array transducer.

3. A method according to claim 1 wherein two receiver coils are used, said two receiver coils being sensitive to acoustic propagation in perpendicular directions.

4. A method according to claim 1, wherein ultrasonic transducer is excited with a sinusoidal voltage for one period of the excitation frequency.

5. A method comprising carrying out the method of claim 1 for a range of frequencies for obtaining multi-frequency images.

6. An apparatus(4) for being used for the application of the method of claim 1, for acquiring information on the electrical conductivity and permittivity distributions in a specimen (2) comprising

a) Static magnetic field generator (8) for generating static magnetic field inside the specimen (2)

*b) an ultrasound transducer (1) for being located on the surface of the specimen (2) and configured to be excited with a sinusoidal voltage of a specific excitation frequency, so as to generate an acoustic wave inside the specimen (2) in the presence of the static magnetic field; and*

*c) a receiver coil (3) for* encircling the specimen (2) or for *being placed near the specimen* (2) *and adapted to detect the Lorentz electric field induced* by the propagation of the acoustic wave Z *inside the specimen, which is a function of the electrical conductivity and permittivity of the specimen*

**Patentansprüche**

1. Verfahren zur Erzeugung von Bildern der elektrischen Eigenschaften einer Probe (2), umfassend:

a) Erzeugen eines statischen Magnetfeldes mittels eines statischen Magnetfeldgenerators (8) innerhalb der Probe (2)

b) in Gegenwart des statischen Magnetfeldes durch Anlegen einer Schallwelle an die Probe (2), indem ein Ultraschallwandler (1), der sich auf der Oberfläche der Probe (2) befindet, mit einer sinusförmigen Spannung einer bestimmten Anregungsfrequenz angeregt wird

c) Erfassen des elektrischen Lorentz-Feldes, das durch die Ausbreitung der akustischen Welle im Inneren der Probe induziert wird, über eine Empfängerspule (3), die die Probe umgibt oder in der Nähe der Probe (2) angeordnet ist

d) Erzeugen von Bildern, die die Leitfähigkeits- und Permittivitätsverteilungen der Probe (2) basierend auf dem detektierten elektrischen Lorentz-Feld zeigen.

2. Verfahren nach Anspruch 1, wobei der Ultraschallwandler (1) ein Einzelelement- oder linearer Phased-Array-Wandler ist.

3. Verfahren nach Anspruch 1, bei dem zwei Empfängerspulen verwendet werden, wobei die beiden Empfängerspulen empfindlich für akustische Ausbreitung in senkrechten Richtungen sind.

4. Verfahren nach Anspruch 1, wobei der Ultraschallwandler für eine Periode der Anregungsfrequenz mit einer sinusförmigen Spannung angeregt wird.

5. Verfahren, umfassend das Durchführen des Verfahrens nach Anspruch 1 für einen Frequenzbereich zum Erhalten von Mehrfrequenzbildern.

6. Vorrichtung (4) zur Verwendung bei der Anwendung des Verfahrens nach Anspruch 1 zum Erfassen von Informationen über die elektrische Leitfähigkeit und Permittivitätsverteilungen in einer Probe (2), umfassend

**a)** Statischer Magnetfeldgenerator (8) zur Erzeugung eines statischen Magnetfeldes innerhalb der Probe (2)
**b)** Ultraschallwandler (1), der sich auf der Oberfläche der Probe (2) befindet und so konfiguriert ist, dass er mit einer sinusförmigen Spannung einer spezifischen Anregungsfrequenz angeregt wird, um in Gegenwart des statischen Magnetfeldes eine Schallwelle innerhalb der Probe (2) zu erzeugen; und
**c)** Empfängerspule (3) zum Umkreisen der Probe (2) oder zum Anordnen in der Nähe der Probe (2) und zum Erfassen des durch die Ausbreitung der Schallwelle innerhalb der Probe induzierten elektrischen Lorentz-Feldes, das eine Funktion der elektrischen Leitfähigkeit und Permittivität der Probe ist.

**Revendications**

1. Procédé de production d'images des propriétés électriques d'un spécimen (2) comprenant :

**a**) Générer via un générateur de champ magnétique statique (8) un champ magnétique statique à l'intérieur du spécimen (2)
**b)** en présence du champ magnétique statique, en appliquant une onde acoustique au spécimen (2), en excitant un transducteur ultrasonore (1) situé à la surface du spécimen (2) avec une tension sinusoïdale d'une fréquence d'excitation spécifique
**c**) détecter par une bobine réceptrice (3) encerclant l'échantillon ou placée près du spécimen (2) le champ électrique de Lorentz induit par la propagation de l'onde acoustique à l'intérieur de l'échantillon
**d**) générer des images montrant les distributions de conductivité et de permittivité du spécimen (2) en fonction du champ électrique de Lorentz détecté.

2. Procédé selon la revendication 1, dans lequel le transducteur ultrasonore (1) est un transducteur à élément unique ou à réseau linéaire en phase.

3. Procédé selon la revendication 1, dans lequel deux bobines de réception sont utilisées, lesdites deux bobines de réception étant sensibles à la propagation acoustique dans des directions perpendiculaires.

4. Procédé selon la revendication 1, dans lequel le transducteur ultrasonore est excité avec une tension sinusoïdale pendant une période de la fréquence d'excitation.

5. Procédé comprenant l'exécution du procédé selon la revendication 1, pour une gamme de fréquences permettant d'obtenir des images multifréquences.

6. Appareil (4) pour être utilisé pour l'application du procédé selon la revendication 1, pour acquérir des informations sur la conductivité électrique et la distribution de la permittivité dans un spécimen (2) comprenant

**a)** Générateur de champ magnétique statique (8) pour générer un champ magnétique statique à l'intérieur du spécimen (2)
**b)** un transducteur ultrasonore (1) pour être placé sur la surface du spécimen (2) et configuré pour être excité avec une tension sinusoïdale d'une fréquence d'excitation spécifique, de manière à générer une onde acoustique à l'intérieur du spécimen (2) en présence du champ magnétique statique ; et
**c**) une bobine réceptrice (3) pour encercler le spécimen (2) ou pour être placée près du spécimen (2) et adaptée pour détecter le champ électrique de Lorentz induit par la propagation de l'onde acoustique à l'intérieur du spécimen, qui est fonction de la conductivité électrique et de la permittivité du spécimen.

Figure 1

Figure 2

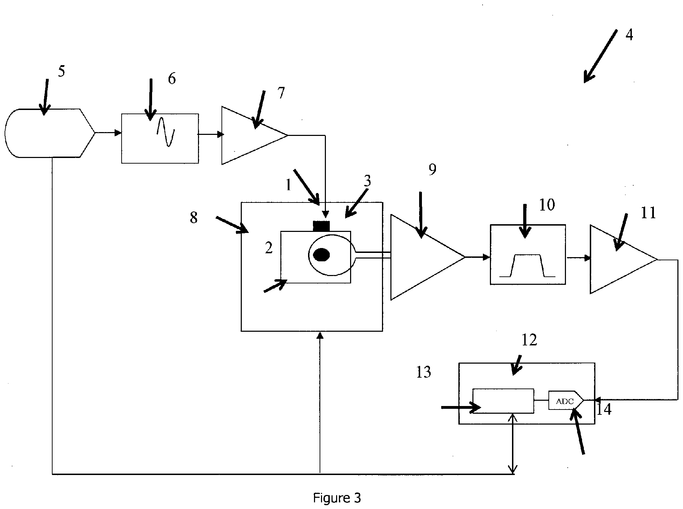

Figure 3

Figure-4

Figure-5

Figure 6

Figure 7

Figure 8

Figure 9

Figure 10

Figure 11

Figure 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9800732 A
- WO 2000019244 A1

- WO 1998000732 A1


**Non-patent literature cited in the description**

- **D. C. BARBER ; B. H. BROWN.** APPLIED POTENTIAL TOMOGRAPHY. *Journal of Physics E-Scientific Instruments,* 1984, vol. 17 (9), 723-733
- **K. PAULSON ; W. LIONHEART ; M. PIDCOCK.** OPTIMAL EXPERIMENTS IN ELECTRICAL-IMPEDANCE TOMOGRAPHY. *Ieee Transactions on Medical Imaging,* December 1993, vol. 12 (4), 681-686
- **P. METHERALL ; D. C. BARBER ; R. H. SMALLWOOD ; B. H. BROWN.** Three-dimensional electrical impedance tomography. *Nature,* April 1996, vol. 380 (6574), 509-512
- Electrical impedance tomography using induced currents. **N. G. GENCER.** Electrical and Electronics Engineering. Middle East Technical University, 1993
- **N. G. GENCER ; M. KUZUOGLU ; Y. Z. IDER.** ELECTRICAL-IMPEDANCE TOMOGRAPHY USING INDUCED CURRENTS. *Ieee Transactions on Medical Imaging,* June 1994, vol. 13 (2), 338-350
- **A.-Z. S ; G. D ; L. G ; Y. Z. Z ; P. A. J ; B. M. S.** *A feasibility study of electromagnetic induction tomography,* 426-9
- **S. AL-ZEIBAK ; D. GOSS ; G. LYON ; Z. Z. YU ; A. J. PEYTON ; M. S. BECK.** *A feasibility study of electromagnetic induction tomography,* 426-9
- **N. G. GENCER ; N. TEK.** *Imaging Tissue Conductivity via Contactless Measuremens: A Feasibility Study,* 1998, vol. 6 (3, http://journals.tubitak.gov.tr/elektrik/issues/elk-98-6-3/elk-6-3-2-98024.pdf
- **H. GRIFFITHS.** Magnetic induction tomography. *Measurement Science & Technology,* August 2001, vol. 12 (8), 1126-1131
- **B. C. TOWE ; M. R. ISLAM.** A MAGNETOACOUSTIC METHOD FOR THE NONINVASIVE MEASUREMENT OF BIOELECTRIC CURRENTS. *Ieee Transactions on Biomedical Engineering,* October 1988, vol. 35 (10), 892-894

- **B. J. ROTH ; P. J. BASSER ; J. P. WIKSWO.** A THEORETICAL-MODEL FOR MAGNETOACOUSTIC IMAGING OF BIOELECTRIC CURRENTS. *Ieee Transactions on Biomedical Engineering,* August 1994, vol. 41 (8), 723-728
- **B. J. ROTH ; J. P. WIKSWO.** Comments on "Hall effect imaging. *Ieee Transactions on Biomedical Engineering,* October 1998, vol. 45 (10), 1294-1295
- **H. WEN ; J. SHAH ; R. S. BALABAN.** Hall effect imaging. *Ieee Transactions on Biomedical Engineering,* January 1998, vol. 45 (1), 119-124
- **H. WEN.** Volumetric Hall effect tomography - A feasibility study. *Ultrasonic Imaging,* July 1999, vol. 21 (3), 186-200
- **A. MONTALIBET ; J. JOSSINET ; A. MATIAS ; D. CATHIGNOL.** Electric current generated by ultrasonically induced Lorentz force in biological media. *Medical & Biological Engineering & Computing,* January 2001, vol. 39 (1), 15-20
- **Y. XU ; S. HAIDER ; A. HRBEK.** *Magneto-acousto-electrical tomography: A new imaging modality for electrical impedance,* 292-295
- **S. HAIDER ; A. HRBEK ; Y. XU.** Magneto-acousto-electrical tomography: a potential method for imaging current density and electrical impedance. *Physiological Measurement,* June 2008, vol. 29 (6), S41-S50
- **Y. XU ; B. HE.** Magnetoacoustic tomography with magnetic induction (MAT-MI). *Physics in Medicine and Biology,* November 2005, vol. 50 (21), 5175-5187
- **X. LI ; Y. XU ; B. HE.** Magnetoacoustic tomography with magnetic induction for imaging electrical impedance of biological tissue. *Journal of Applied Physics,* March 2006, vol. 99 (6
- **R. M. XIA ; X. LI ; B. HE ; IEEE.** *Magnetoacoustic tomography of biological tissue with magnetic induction,* 2007
- **K. BRINKER ; B. J. ROTH.** The effect of electrical anisotropy during magnetoacoustic tomography with magnetic induction. *Ieee Transactions on Biomedical Engineering,* May 2008, vol. 55 (5), 1637-1639

- **R. PLONSEY ; R. COLLIN.** Principles and Applications of Electromagnetic Fields. Mc.Graw-Hill, 1961
- **M. CARLEY.** *Some Notes on Acoustics,* 2001
- **T. G. ZIELINSKI.** Fundamentals of multiphysics modelling of piezo-poro-elastic structures. *Archives of Mechanics,* 2010, vol. 62 (5), 343-378
- **J. R. MORTARELLI.** A GENERALIZATION OF THE GESELOWITZ RELATIONSHIP USEFUL IN IMPEDANCE PLETHYSMOGRAPHIC FIELD CALCULATIONS. *Ieee Transactions on Biomedical Engineering,* 1980, vol. 27 (11), 665-667